(19) Europäisches Patentamt
European Patent Office
Office européen des brevets

(11) Publication number: 0 201 105 B1

(12) **EUROPEAN PATENT SPECIFICATION**

(45) Date of publication of patent specification: **28.02.90**

(51) Int. Cl.$^5$: **C 07 C 67/40**, C 07 C 69/14, C 07 C 69/24

(21) Application number: **86106352.7**

(22) Date of filing: **09.05.86**

(54) **A method to convert primary alkanols to their corresponding esters.**

(30) Priority: **10.05.85 US 732835**

(43) Date of publication of application: **12.11.86 Bulletin 86/46**

(45) Publication of the grant of the patent: **28.02.90 Bulletin 90/09**

(84) Designated Contracting States: **DE FR GB IT SE**

(56) References cited:
**EP-A-0 151 886**
**GB-A- 287 846**
**GB-A- 312 345**
**GB-A- 470 773**

(73) Proprietor: **UNION CARBIDE CORPORATION**
**39 Old Ridgebury Road**
**Danbury Connecticut 06817 (US)**

(72) Inventor: **Keen, Brian Terry**
**1528 Autumn Road**
**Charleston West Virginia 25314 (US)**

(74) Representative: **Wuesthoff, Franz, Dr.-Ing. et al**
**Patentanwälte Wuesthoff -v. Pechmann-Behrens-Goetz Schweigerstrasse 2**
**D-8000 München 90 (DE)**

EP 0 201 105 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Brief summary of the invention

1. Technical field

This invention relates generally to the conversion of primary alkanols having a carbon chain between $C_1$ and about $C_{15}$ to their corresponding.

2. Background art

The commercial preparation of esters has traditionally required a two step process. The process involves oxidation of the alcohol to an acid followed by the reaction of the acid with another alcohol to form the desired ester. The resulting ester can have many commercial uses. Lower molecular weight esters are valuable as solvents. Lower molecular weight esters can also be used as intermediates to form corresponding acids, for example acetic acid. Higher molecular weight esters can be used as lubricants or intermediates in the preparation of elastomers and synthetic fibers.

The direct or indirect usefulness of esters in commercial products has generated interest in methods for the inexpensive development of esters. The development of these methods has centered on either the reduction in the number of steps to produce the ester, a decrease in the expense of the reactants and catalyst utilized in the methods, or ways of increasing the total ester yield of the methods. The following patents are examples of methods developed for the production of esters.

US—A—4,052,424 discloses a process for the production of esters from alkanols. This process achieves a high yield of esters by contacting alkanols in vapor phase with a silver-cadmium alloy catalyst. In this process the alkanol is vaporized either alone or in an admixture of nitrogen, hydrogen, carbon monoxide, or carbon dioxide. The vapor is passed through a reaction zone containing the silver-cadmium alloy catalyst. The catalyst can be a fixed, moving, or fluidized bed. Tha reaction zone temperatures are between about 250°C and about 600°C. The reaction zone pressure can vary in a range between about 5 and about 2000 psia. The admixture of gases with the alkanol vapor is used to control undesirable side reactions.

US—A—3,639,449 discloses a process for the manufacture of esters of carboxylic acids. This method involves reacting at least one alcohol in a mixture of at least one alcohol and at least one aldehyde with molecular oxygen in the presence of a catalyst. The catalyst is selected from the metals of Group VIII of the Periodic Table having an atomic number from 44 to 78, oxides of these metals, pretreated products of these metals, and pretreated products of these metal oxides, with the proviso that when alcohols alone are reacted with molecular oxygen, at least one primary alcohol should be present in the alcohols. The method disclosed in this patent uses a palladium chloride catalyst and generates hydrogen gas chloride after the end of the reaction. The reduced palladium is deposited out of the system and regeneration of the catalyst is necessary. Regeneration occurs with copper chloride or copper acetate which oxidizes the catalyst back to palladium chloride.

US—A—3,452,067 discloses a method for producing esters from alcohols. This process prepares esters by catalytic dehydrogenation and disproportionation of alcohols, in vapor phase, in the presence of molybdenum sulfide catalyst. The resulting esters contain twice as many carbon atoms as the orginal alcohols. The reaction occurs from 400°F to 600°F over a supported catalyst. The process of this patent uses aliphatic, saturated, primary alcohols containing between 2 and about 15 carbon atoms. This process can use mixtures of primary alcohols to obtain esters of uneven carbon chain lengths extending from the two sides of the ester functional group. The catalyst carriers can include various aluminous and silicious materials.

US—A—2,079,414 discloses a process for producing alcohols from esters of non-aromatic carboxylic acids. In this process an ester of a non-aromatic carboxylic acid is treated with hydrogen gas at a temperature in excess of 250°C and in a superatmospheric pressure. This reaction is conducted in the presence of a hydrogenation catalyst. The catalyst is identified as being either a single hydrogenating metal or its oxide or a mixture of hydrogenating metals and their oxides. Examples of catalysts include those containing copper nitrate, copper oxide, copper chromite, and others.

From GB—A—470,773 the manufacture of esters, particularly of ethyl acetate has been known, in which a primary alcohol to be converted is passed over a dehydrogenation catalyst in the form of a reduced metal, at a temperature of from 200°C to 260°C, optionally containing a promoter in the form of oxides of titanium, thorium or uranium. From said printed publication it has moreover been known that if a substantially equimolecular mixture of alcohol and hydrogen is passed over catalysts consisting of magnesium oxide and other oxides between 220°C and 350°C, the formation of higher alcohols, especially butanols and hexanols in addition to acetaldehyde, butyraldehyde and crotonaldehyde is observed. Many of the processes used in the industry to form esters from an alkanol and in particular ethanol require an essentially pure alkanol feed solution. In processes which convert ethanol, commercial grade 190 proof ethanol is not suitable as an alkanol feed solution. This is because the 5 percent water concentration in the alkanol feed solution decreases the productivity of the resulting ester. Processes that cannot use aqueous alcohols require the feed alcohols to be dried to pure 200 proof ethanol. Pure ethanol can cost from 5 to 20 cents per gallon more than the commonly available 190 proof ethanol. In large scale production of esters, this price difference can have a significant effect on the price of the resulting esters.

The industry lacks a method to produce esters from primary alkanols wherein the reaction has a high selectivity conversion to the desired esters and a low formation of by-products such as acids, aldehydes, higher alcohols, and gaseous derivatives. Additionally, the industry lacks a method that can efficiently use aqeuous alkanol feed solutions.

3. Disclosure of the invention

This invention includes a method for producing an ester by reacting a primary $C_1$—$C_{15}$ alkanol in vapor phase at a temperature of 200 to 300°C and a pressure of 0 to 34.5 bar in the presence of a dehydrogenation catalyst, and recovering the ester wherein the catalyst is a copper chromite catalyst, optionally containing barium oxide and the mole ratio of hydrogen and alkanol is maintained in the range of 0.4 to 5 moles $H_2$, preferably 0.6 to 3 moles $H_2$ to 1 mole alkanol.

The invention can include collecting and then separating alkanoate ester, water, and alkanol by a means for azeotropically distilling the reactor effluent. When an azeotropic distillation step is included with the method of this invention, the anhydrous alkanol that is separated can, optionally, be recycled as alkanol feed solution. The distilled alkanol can be mixed with an aqueous alkanol feed solution without a significant decrease in productivity or efficiency of ester production.

Brief description of the drawings

Figure 1 is a block diagram of an alkonal conversion unit including a means for azeotropically distilling the reactor effluent suitable for use with the method of this invention on an industrial scale.

Figure 2 is a schematic diagram of a continuous ester production unit without a means for azeotropically distilling the reactor effluent suitable for forming esters from alkanols on a pilot plant scale.

Within the reactor, the mole ratio of hydrogen gas to alkanol feed solution is selectively regulated to optimize the reaction equilibrium to favor the production of esters and suppress the formation of undesirable by-products. Regulating the mole ratio of hydrogen gas can be accomplished by a means for controlling the rate of flow of hydrogen gas, the reactor temperature, the reactor pressure or a combination of these. The rate of flow of hydrogen gas, reactor temperature, and reactor pressure are controlled to drive the reaction equilibrium towards a high productivity and high efficiency in the formation of the desired esters. The reaction products including the formed esters are then removed from the reactor. The esters can be further purified by a means for azeotropic distillation. When an azeotropic distillation step is used, anhydrous alkanol can be recycled as alkanol feed solution to the reactor.

An efficient, commercially practical, and direct process for conversion of anhydrous or aqueous alkanols, having carbon chains of $C_1$ to $C_{15}$, to their corresponding esters involves the following chemical process steps. The first reaction to occur in the presence of the catalyst is a quickly established equilibrium between the alkanol and its aldehyde:

$$R_1CH_2OH \leftrightarrows R_1\overset{\overset{\displaystyle O}{\|}}{C}H + H_2 \qquad (1)$$

wherein, $R_1$ is a member selected from hydrogen or an alkyl group of up to 14 carbon atoms. This reaction is governed by the following equilibrium constant:

$$K=\frac{[R_1\overset{\overset{\displaystyle O}{\|}}{C}H]\ [H_2]}{[R_1CH_2OH]}.$$

The second reaction to occur is the formation in equilibrium of the hemiacetal from the alkanol and aldehyde:

$$R_1\overset{\overset{\displaystyle O}{\|}}{C}H + R_1CH_2OH \leftrightarrows R_1\overset{\overset{\displaystyle OH}{|}}{C}HOCH_2R_1. \qquad (2)$$

The primary rate limiting step in the conversion reactions of an alkanol to its corresponding ester is the reaction from the hemiacetal to the ester. This reaction is an equilibrium that greatly favors the formation of the ester:

$$R_1\overset{\overset{\displaystyle OH}{|}}{C}HOCH_2R_1 \leftrightarrows R_1\overset{\overset{\displaystyle O}{\|}}{C}OCH_2R_1 + H_2. \qquad (3)$$

The net reaction equation is:

$$\text{(net)}\quad 2R_1CH_2OH \rightleftharpoons R_1CH_2O\overset{O}{\overset{\|}{C}}R_1+2H_2.$$

It is hypothesized from this series of reactions, that the rate of formation of the ester is dependent upon the concentration of hemiacetal:

Rate of ester formation=k×[hemiacetal].

The rate of formation of the hemiacetal is dependent upon the concentrations of both the aldehyde and alkanol:

Rate of hemiacetal formation=k×[aldehyde][alkanol].

The formation of an alkanol to its corresponding ester is a first order relationship to the concentration of the aldehyde intermediate compound.

In alkanol conversion reactions using copper containing catalysts the most significant by-products are from condensation of the aldehyde intermediate compound. The aldehyde intermediate compound is in equilibrium with its corresponding aldol:

$$(4)\quad 2R_1CH_2\overset{O}{\overset{\|}{C}}H \rightleftharpoons R_1CH_2\overset{OH}{\overset{|}{C}}H\underset{R_1}{\underset{|}{C}}H\overset{O}{\overset{\|}{C}}H.$$

The aldol compound is in equilibrium with an unsaturated alcohol and water:

$$(5)\quad R_1CH_2\overset{OH}{\overset{|}{C}}H\underset{R_1}{\underset{|}{C}}H\overset{O}{\overset{\|}{C}}H \rightleftharpoons R_1CH_2CH{=}\underset{R_1}{\underset{|}{C}}\overset{O}{\overset{\|}{C}}H+H_2O.$$

The unsaturated alcohol in the presence of hydrogen gas irreversibly forms a branched aldehyde:

$$(6)\quad R_1CH_2CH{=}\underset{R_1}{\underset{|}{C}}\overset{O}{\overset{\|}{C}}H+H_2 \rightarrow R_1CH_2CH_2\underset{R_1}{\underset{|}{C}}H\overset{O}{\overset{\|}{C}}H.$$

The net by-product equation is:

$$\text{(net)}\quad 2R_1CH_2\overset{O}{\overset{\|}{C}}H+H_2 \rightleftharpoons R_1CH_2CH_2\underset{R_1}{\underset{|}{C}}H\overset{O}{\overset{\|}{C}}H+H_2O.$$

The formation of an aldehyde to its by-products is a second order relationship or the square of the concentration of the aldehyde compound. Therefore, the selectivity of efficiency of an alkanol conversion unit having a copper containing catalyst is significantly dependent on the averagk concentration of aldehyde in the reaction zone. This is because the by-products of aldehyde are more affected by the concentration of aldehyde than is the formation of the corresponding ester. An increase in the reactor zone of the concentration of aldehyde, when a copper containing catalyst is present, does not significantly alter the concentration of the resulting ester. When zinc is present in a catalyst the formation of an ester from an alkanol is dependent upon the concentration of aldehyde in the reactor zone.

The method of this invention has the unexpected result of increasing the concentration of the ester in an alkanol conversion reaction by regulating and increasing the presence of hydrogen gas in the reaction zone. This result is unexpected for two reasons.

The first reason that the results of the method of this invention are unexpected is that the addition of hydrogen gas depresses or partially inhibits the formation of the aldehyde intermediate compound of reaction equation (1). The formation of the aldehyde intermediate compound is partially inhibited by the increased concentration of hydrogen gas because of the interrelationship of these two compounds as

depicted in the equation of their equilibrium constant. The aldehyde intermediate compound is required for the formation of the corresponding ester. The suppression of the aldehyde intermediate compound, by regulating the concentration of hydrogen gas, increases the selectivity of the aldehyde for the formation of the corresponding ester by decreasing its availability for by-product formation.

The second reason that the results of the method of this invention are unexpected is that hydrogeneration of an ester to its corresponding alcohol is a well known process. The presence of an increased concentration of hydrogen gas can be expected to suppress the formation of the ester in reaction equation (3). By regulating the presence of hydrogen gas in the reactor zone according to this invention the formation of the ester is enhanced. A concentration of hydrogen gas in the reactor zone above that sufficient to regulate the reaction equilibrium between the primary alkanol and the aldehyde intermediate compound does decrease the formation of the corresponding ester compound.

The regulating of a mole ratio hydrogen gas to a primary alkanol within a reactor and, thus the reactor zone, can be accomplished by numerous means for controlling the rate of flow of hydrogen gas, the reactor temperature, the reactor pressure, or combinations of these. The regulating of hydrogen gas in a reactor can be from an exogenous supply source or more preferably by recycling separated hydrogen gas from the reaction products. The mole ratio of hydrogen gas to primary alkanol can also be regulated by feeding aldehyde to the reactor.

As can be observed from the above equations, an increase in temperature has the effect of increasing the equilibrium constant. An increase in the pressure at which the reaction occurs decreases the relative amount of aldehyde formed an intermediate compound. An increase in the concentration of hydrogen gas then decreases the concentration of aldehyde. It has been found that the concentration of aldehyde present during the reaction, to further react with the alkanol and form the desired alkanoate ester, is dependent upon the rate of the reaction, but is controlled by the equilibrium of the reaction. By regulating the concentration of hydrogen gas in the reactor the concentration of aldehyde present during the reaction is controlled. The regulation of the mole ratio of hydrogen gas to alkanol feed solution within the reactor according to this invention partially inhibits the formation of the aldehyde intermediate compound and enhances the selectivity of the aldehyde for the formation of an alkanoate ester reaction product.

The major inefficiency encountered with the conversion reaction of a primary alkanol to its corresponding ester is the formation of aldehyde condensation products. For example, in the ethanol to ethyl acetate process, the condensation products of acetaldehyde are crotonaldehyde, butyraldehyde, and butanol. Crotonaldehyde forms by dehydration of acetaldol (acetaldehyde dimer). The rate of formation of crotonaldehyde is dependent on the square of the acetaldehyde concentration. The ethyl acetate ester rate of formation is dependent on the acetaldehyde concentration to the first order. The alkanoate ester production efficiency is directly related to the degree of aldehyde condensation and is directly dependent on the aldehyde concentration in the reaction zone. As can be understood from these relationships, a reaction method which regulates the mole ratio of hydrogen gas to moles of alkanol feed solution in reaction zone results in an enhanced formation of alkanoate esters. The control of the mole ratio of hydrogen gas to alkanol feed solution by regulating hydrogen gas feed rate, reactor temperature, and reactor pressure, allow one to selectively control and optimize the conversion reaction of alkanols to their corresponding alkanoate esters.

The regulation of the mole ratio of hydrogen gas to the alkanol feed solution in the reaction zone must be sufficient to suppress the concentration of aldehyde. Preferably, the mole ratio of hydrogen gas to alkanol feed solution in the reaction zone is such that the concentration of aldehyde is decreased sufficiently to increase the efficiency of ester production. The mole ratio of hydrogen gas should not be so high as to essentially, completely inhibit the formation of the aldehyde intermediate compound. Too high of a concentration of hydrogen gas in the reaction zone can suppress the presence of aldehyde and have an adverse affect on the conversion of alkanol to alkanoate ester as discussed above.

Inhibiting the concentration of aldehyde present in the reaction zone increases the efficiency of the reaction, but the total amount of alkanoate ester produced is decreased. The most preferred mole ratio of hydrogen gas to alkanol feed solution in the reaction zone is 0.6 mole to 3 moles of hydrogen gas to 1 mole of alkanol feed solution A desirable ratio of moles of hydrogen gas to moles of alkanol feed solution is 0.4 mole to 5 moles of hydrogen gas to 1 mole of akanol feed solution. A mole ratio of hydrogen gas to alkanol feed solution of trace amounts of hydrogen gas such as 0.05 mole or less than 12 moles of hydrogen gas to 1 mole alkanol feed solution is effective. The suppression of ester formation starts to become adverse to the conversion reaction above about 12 moles of hydrogen gas to 1 mole of alkanol feed solution.

The method of this invention is a vapor phase process and can employ one of many commercially available copper chromite containing catalysts. The process of this invention can operate well with these catalysts in a range of reactor temperatures between 200°C and 300°C and reactor pressures between 0 bar (psig) and 34.5 bar (500 psig). More preferred temperature and pressure ranges are between 220°C and 260°C and between 0.69 bar (10 psig) and 6.9 bar (100 psig). respectively. The most preferred reactor pressure is between 2.07 bar (30 psig) and 5.52 bar (80 psig). Above 300°C to 350°C the addition of increased hydrogen gas to the reactor increases the efficiency of ester production, but also increases by-product formation. The mole ratio of hydrogen gas to alkanol feed solution required to enhance ester production can vary with reactor temperature. An increase in reactor temperature requires an increase in the moles per hour of hydrogen gas needed to increase ester efficiency.

The method of this invention provides a high alkanol conversion of typically between 20 and 75 percent. The production of alkanoate ester, when performed by the method of this invention, results in an efficiency of 80 and 100 percent. This method has a corresponding productivity of between 160—800 $kg/m^3 \cdot h$ (10 to 50 pounds of ester per cubic foot of catalyst hour). In addition to the high conversion efficiency and productivity, this process produces a large amount of valuable, high-quality hydrogen gas which is usable in other chemical processes.

The copper chromite containing catalysts used by the method of this invention can operate for prolonged periods of time. These catalysts can be regenerated and are less expensive than exotic metal catalysts. The prolonged operationg periods for copper chromite catalysts used in alkanol conversion reactions can exceed 6000 to 8000 hours of operation per year and can be used for four or more years. An 8000 hour operation period equates to roughly one year of typical commercial use of a catalyst. The prolonged periods of operation of copper chromite containing catalysts are due in part to the resistance of copper chromite to organic acids inherent in the process. When organic acids are in contact with certain catalysts, particularly those containing zinc, catalyst can be destroyed. The prolonged use of a catalyst to conduct this reaction can cause the build-up of carbon deposits within the catalyst. This results in a loss of activity in the catalyst. Copper chromite containing catalysts can be regenerated by stopping the feed of hydrogen gas and alkanol feed solution to the reactor and feeding air through the reactor. The air "burns off" the carbon deposits and reestablishes the activity of the catalyst to its initial level. The regeneration properties of copper chromite catalysts are desirable when compared to catalysts in which the deactivation by carbon deposits is irreversible. Copper chromite catalysts are typically regenerated 5 to 6 times a year and a total of about 20 times during their operational period without loss of activity. The expense of copper chromite catalysts is less than certain other catalysts, particularly those that contain silver.

Copper chromite catalysts suitable for use with this invention are supplied by the Harshaw/Filtrol Company, 1945 East 97th Street, Cleveland, Ohio 44106. The copper chromite catalyst used in the most preferred embodiments of this invention is provided by this source and is designated Harshaw Cu-0203. Other copper chromite catalysts and catalysts having a mixture of copper and other materials such as alumina can be obtained and used with this invention. Specific, desirable examples include the E-113 TU catalyst from the Mallinckrodt Corporation, Calsicat Division, 1707 Gaskell Avenue, Erie, PA 16503, and the G-13 or G-22 catalysts from the United Catalysts Corporation, P.O. Box 32370, Louiville, KY 40232. The method of this invention is not limited to a particular copper chromite containing catalyst. Suitable catalyst can contain other catalytic metals such as barium. The efficiency of the reaction in the method of this invention is not dependent on the catalyst, but upon the mole ratio of hydrogen gas to the alkanol feed solution in the reaction zone. It is the control of the equilibrium of the reaction and not the catalyst that increases the efficiency of the reaction of this method. Specific examples of copper containing catalysts are:

Harshaw catalysts

| | |
|---|---|
| Non-barium containing catalysts | |
| Cu-0203 | |
| Cu-1422 | |
| Cu-1808 | |
| Cu-1809 | |
| Barium oxide containing catalysts | Barium oxide |
| Cu-1107 | 7% |
| Cu-1117 | 8% |
| Cu-1132 | 9% |
| Cu-1184 | 9% |
| Cu-1186 | 9% |
| Cu 2501G410 (granules) | 5% Cu on silica |

Calsicat corporation

| | |
|---|---|
| Non-barium containing catalysts | |
| E-103 Tu | |
| E-105 | |
| E-108 | |
| E-113 TU | |
| E-403 TU | |
| Barium oxide containing catalysts | Barium |
| E-101 | 3—7% |
| E-102 | 3—7% |
| E-106 TU | 8% |
| E-406 TU | 8% |

United catalysts corporation

Non-barium containing catalysts
G13 3% Manganese 39%Cu 37% Chrome (metal not oxide)

Barium oxide containing catalysts
G22 10—11% Barium

The alkanoate esters obtained by the method of this invention can be further separated and purified by collecting the effluent from the reactor and separating the desired products by azeotropically distilling the effluent. The reaction products in the effluent include alkanoate ester, water, and anhydrous alkanol are and other by-products formed during the reaction. The separated anhydrous alkanol can be recycled to the alkanol feed solution to the reactor. By recycling the separated anhydrous alkanol, the method according to this invention, can productively use aqueous alkanols as alkanol feed solutions. For example, in the conversion of ethanol to ethanoate ester or ethyl acetate, commercial grade 190 proof (95 weight percent) ethanol can be used as the alkanol feed solution. To use 190 proof ethanol as a feed solution, typically, requires the reaction to be initiated with 200 proof ethanol. The reaction products must be formed in sufficient concentrations to begin azeotropic distillation. The anhydrous ethanol is then recycled and mixed with the 190 proof ethanol feed solution. Sufficient distilled, anhydrous alkanol can be produced to form a 1:1 mixture with the aqueous or 190 proof ethanol. The mixture has an effective concentration of about 2.5 percent water. The continued distillation and recycling of anhydrous ethanol permits the sole use of 190 proof ethanol as a feed solution in this method after initiation of the reaction.

The alkanol to alkanoate ester conversion productivity is dependent on the water content of the alkanol feed solution. By raising the reaction temperature and maintaining a sufficient mole ratio of hydrogen gas to regulate the equilibrium of the reaction of this invention, some of the productivity loss from the presence of water in the alkanol feed solution can be restored. The productivity loss due to water in the alkanol feed solution remains evident when compared to the results obtained with a pure alkanol feed solution, but the resulting level of ester production is commercially acceptable. This is because of (1) the cost differential between aqueous alkanol feed solutions and pure alkanol feed solutions or (2) the expense of drying an aqueous alkanol feed solution is greater than the economic loss incurred by the lower productivity of alkanoate ester.

Fermentation ethanol feed solutions can also be used with the method of this invention. Fermentation ethanol feed solutions are less pure than commercial grade 190 proof ethanol. The additional water concentration of fermentation ethanol feed solutions decrease the productivity of the reaction of this method. This method provides an additional commercial use for fermentation ethanol.

An industrial scale unit suitable for conducting the method of this invention can be further understood by reference to Figure 1. An alkanol feed tank 1 or feed source supplies alkanol feed solution through a feed line 10 to a reactor 2. The reaction products or effluent exit the reactor 2 through a line 11 and enter a hydrogen gas separator 3. Hydrogen gas is removed from the system and transported through a hydrogen gas line 12 and either recirculated into the feed line 10, through a means for recycling hydrogen gas such as a line 22 and a hydrogen compressor 9, or to a hydrogen gas collector 4. Various suitable means for controlling or regulating the recirculated hydrogen gas such as automatic or manual control values and regulators can be used on lines 10 and 12 to regulate the mole ratio of hydrogen gas in the reactor 2 with the recirculated hydrogen gas. The liquid product or effluent of the reaction exiting the hydrogen gas separator 3 travels via a liquid product line 13 to a means for azeotropically distilling the reactor effluent or a removal column 5. From the top section of the removal column 5, ethers, aldehydes, and low molecular weight by-products or "lights" are recycled through a line 14 to the feed tank 1. From a lower section of the removal column 5, anhydrous alkanol is removed and transported via line 15 back to feed line 10. A purge line 22 removes "heavies" or higher moleculur weight organic by-products from the system to line 18. From the middle section of the removal column 5, the alkanol, water, and alkanoate ester azeotrope are removed via a line 16 and transported to a means for collecting effluent or a water decanter 6. Make-up water can be supplied to the water decanter via line 16 from a make-up water supply source 7 attached to a line 16 by a make-up water supply line 17. The aqueous layer is removed from the water decanter through a line 18 and taken to an organic recovery column (not shown). The top layer from the water decanter 6 consists of concentrated alkanoate ester. Typically, this effluent is 93 percent or higher in ester, about 5 percent alkanol, and about 2 percent or less water. This mixture is transported via a line 19 to an alkanol separation column 8. Purified anhydrous ester is removed from the column through a line 20 and is transported to a storage container or recycled to the feed line 10 (not shown). A mixture of alkanol, water, and alkanoate ester azeotrope is recycled through line 21 back to a line 16 for further separation.

This invention is further understood from the following examples. Abbreviations used in the data and description of these examples are defined as follows:

| Abbreviation | Definition |
|---|---|
| AcH | acetaldehyde |
| AcOH | acetic acid |
| Acet | acetone |
| BuAl | butyl aldehyde |
| BuOH | butanol |
| Eff | efficiency |
| $Et_2O$ | ether |
| EtOAc | ethyl acetate ester |
| EtOH | ethanol |
| IBuOH | isobutanol |
| LHSV | liquid hourly space velocity |
| misc | miscellaneous |
| PrOH | propanol |
| Yr | year |

Experimental procedures

The apparatus and procedure for conducting the following examples is further understood with reference to Figure 2. The apparatus displayed in Figure 2 reacts an alkanol in the vapor phase by contacting it with a copper chromite catalyst in a reactor. Not shown in this figure is the apparatus for azeotropically distilling the reactant products for further purification. The apparatus displayed in Figure 2 is suitable for conducting the method of this invention on a pilot plant scale.

The equipment illustrated in Figure 2, which is used to conduct the method of this invention, includes a reactor 30. The reactor 30 has 25.4 mm (one inch) inside diameter and has a 1.2 m (48 inch) long hot tube with a Dowtherm jacketed lining. Within the reactor 30 is a preheat section 31 containing glass beads, a catalyst bed 32, and a support section 33 containing glass beads. A nitrogen cylinder 35 and a hydrogen gas cylinder 36 supply their respective gases through a gas feed line 37. Attached to this gas feed line can be related apparatuses such as rotometers 25a and 25b, metering valves 26a and 26b, and check valves 27a and 27b. These rotometers and valves assist in the operation and safety of the reaction and are means for regulating the mole feed ratio of hydrogen gas to alkanol feed solution in the reactor. The nitrogen and hydrogen gases passing through the gas feed line 37 pass through a pressure gauge 38 before entering the reactor 30.

A 15 to 19 l (4 to 5 gallon) feed tank 40 supplies alkanol feed solution through a supply line 41. The feed solution is passed from a feed line 41 to a feed line 42 by a pump 43. The feed line 42 is wrapped with heat tape. The tank 40 and the heated supply line 42 are the supply source to the reactor 30 of primary alkanol feed solution in vapor phase. Recycled alkanol is cooled and collected in a feed tank 44 and resupplied to the system through a feed line 45 which has a water cooled jacket. The contents of the feed line 45 enter a feed line 41. The alkanol from the feed line 42 enter the reactor 30, and come in contact with the hydrogen gas from the gas feed line 37. The alkanol feed solution and hydrogen gas are contacted with the catalyst bed 32. The alkanol esters and other reaction products exit the reactor 30 through an exit line 50 which has a reactor pressure gauge 51 and pressure regulator 52. The reaction products pass through a water cooled condenser 53 and are collected in a product receiving tank 54. The product is further passed through a vented ice trap 55 and an unvented ice trap 56. The condensate product is collected and the hydrogen gas is collected through a line 57 in a pressure trap 58.

An emergency shut down system is provided by shut down device 60 which controls the reactor variac 65 and shut down device 61 which controls the feed pump and monitors the temperature of the product condenser. Other gauges, regulators, valves, and related equipment as depicted in Figure 2 can be incorporated into the conversion unit and are within the skill of those in the art.

The numbered examples are examples illustrating this invention. The lettered examples are for comparative purposes.

Example 1

This example demonstrates the effect of hydrogen gas on the conversion reaction of ethanol to ethyl acetate using the equipment and procedure described above. Ethanol in vapor phase is contacted with 180 cc of Harshaw/Cu-0203 copper chromite catalyst. The catalyst was in continuous operation for over 1000 hours and was regenerated prior to obtaining the results in Table 1. The results of regulating the mole ratio of hydrogen gas to alcohol feed are in Table 1.

TABLE 1
Effect of regulating hydrogen gas on ethanol to ethanoate reaction[1]

| LHSV[2] per hour | $H_2$ (Mole/h) | Moles $H_2$ per mole of ethanol feed | Acetaldehyde[3] weight % | Product total ethyl acetate | |
|---|---|---|---|---|---|
| | | | | Weight % | Efficiency %[4] |
| 0.8 | 0.4 | 0.16 | 2.8 | 24.6 | 86 |
| 0.6 | 0.8 | 0.43 | 2.0 | 27.6 | 89 |
| 0.6 | 1.5 | 0.80 | 1.6 | 27.9 | 93 |
| 0.6 | 3.0 | 1.60 | 1.1 | 25.2 | 95+ |

[1] The reaction temperature and pressure to obtain these results was 231°C and 4.14 bar (60 psig), respectively.
[2] LHSV represents the volume of liquid feed per volume of catalyst hour. A value between 0.6 and 1.0 is commercially acceptable.
[3] A product total of acetaldehyde of about 1 percent is considered commercially desirable.
[4] Efficiency percent is the percent ratio of the weight percent of alkanoate ester to the weight percent of the total reaction products including water.

The results of this example demonstrate that the control of the mole ratio of hydrogen gas to alcohol feed with respect to hydrogen gas feed rate, reaction temperature, and reactor pressure is necessary to keep the intermediate aldehyde concentration low throughout the reaction zone. As the moles per hour of hydrogen gas were increased to about 3 moles per hour for a 0.6 LHSV the product total of aldehyde was reduced to about 1 percent. At 1 percent acetaldehyde concentration in the production of ethyl acetate ester provides a commercially desirable result of at least a 95 efficiency percent of ethyl acetate and a corresponding decrease of other condensation products of acetaldehyde.

A 0.8 moles per hour hydrogen gas in this example represents a 0.43:1 ratio of moles of hydrogen gas to moles of ethanol feed solution, respectively. A 3.0 moles per hour hydrogen gas represents a 1.6:1 ratio of moles of hydrogen gas to moles of ethanol respectively.

Example 2

In this example the effect of water in the feed solution of alkanol is demonstrated. In this example, ethanol is converted to ethyl acetate by the same procedure used in Example 1. This example demonstrates that ethanol to ethyl acetate conversion productivity is dependent on the water content of the feed solution. The results of this example are presented in Table 2.

TABLE 2
Effect of water in the feed[1]

| % $H_2O$ in feed ethanol | Acetic acid weight % | Ethyl acetate | |
|---|---|---|---|
| | | Weight % | Efficiency % |
| 0 | 0.04 | 26.3 | 93.1 |
| 1 | 0.07 | 17.3 | 92.7 |
| 3 | 0.11 | 11.3 | 92.5 |
| 5 | 0.17 | 9.4 | 92.8 |

[1] In this example the LHSV was 0.6, temperature was 231°C, pressure was 4.14 bar (60 psig) and hydrogen gas was maintained at 1.5 mole/h, or 0.8 mole of hydrogen gas to mole of alcohol feed.

The results of the example demonstrate that the water content in the ethanol feed solution directly effects the weight percent of the ethyl acetate production. An increase in the amount of water in the ethanol feed solution thereby lowers the conversion of ethanol to the desired product.

Of note is the correlation between this example and Example 1 of the efficiency of ester formation to the mole feed ratio of hydrogen gas to alcohol feed.

Example 3

In this example, ethanol is converted to ethyl acetate by the same procedure used in Example 1. In this example the effect of water in the feed solution and elevated temperatures of the reaction are demonstrated. The decrease in ethanol to ethyl acetate conversion productivity demonstrated in Example 2 can be partially compensated for by elevating the reaction temperature. Additionally, this example projects the productivity that can be expected by conducting the method of this invention on an industrial scale. The results of this example are demonstrated in Table 3.

TABLE 3

Effect of water in the feed solution elevated temperature[1]

| Reactor hours of operation | LHSV $h^{-1}$ | Feed $H_2O$ weight % | Acetic acid weight % | Ethyl acetate wt% | Eff% | Productivity of ethyl acetate[2] $kg/m^3 \cdot h$ (lbs/cb.ft/h) | t/Yr | (MM#/Yr) |
|---|---|---|---|---|---|---|---|---|
| 2000 | 1.0 | 0 | 0.17 | 40.3 | 90.8 | 320 (20) | 54026 | (119) |
| 2023 | 1.0 | 2 | 0.28 | 27.2 | 90.1 | 217.6 (13.6) | 36320 | (80) |
| 2048 | 0.6 | 4 | 0.49 | 29.4 | 90.7 | 140.8 (8.8) | 23608 | (52) |

[1] In this example temperature was 250°C, the pressure was 4.14 bar (60 psig), and hydrogen gas was maintained at 2.0 mole/h.

[2] These projections assume the use of a standard 21.2 $m^3$ (750 cubic) food converter operated for 8000 hours per year. The symbol, MM#/Yr, is an abbreviation for millions of pounds of ethyl acetate per year.

This example demonstrates that the method of this invention can effectively utilize inexpensive aqueous ethanol (190 proof) and achieve a commercially desirably efficiency percentage. The projection of commercial results based on this productivity and the use of a standard converter for one year are also presented.

Of note is the correlation between this example and example 1 of the efficiency of ester formation to the mole feed ratio of hydrogen gas to alcohol feed.

Example 4

This example demonstrates the selectivity of the method of this invention for the conversion of ethanol to ethyl acetate. In this example, ethanol is converted to ethyl acetate by the same proceduce used in Example 1. In this example 180 $cm^3$ of 3.175 mm Harshaw Cu-0203 catalyst is used after about 900, 1400, and 1500 hours of operation to obtain the reactions products of Tables 4, 5, 6, respectively. A 200 proof ethanol feed solution was used to obtain the products of Tables 4 and 5. A 190 proof ethanol feed solution was used to obtain the products of Table 6. The analysis of the reaction products is presented in Tables 4 through 6.

TABLE 4

| Products* | Weight % | Selectivity |
|---|---|---|
| Ethyl acetate | 42.3 | 82.8 |
| Acetic acid | 0.5 | 1.0 |
| Acetaldehyde | 3.2 | 6.3 |
| Butanol+butanal | 4.0 | 7.8 |
| Ethyl ether | 0.9 | 1.8 |
| Acetone | 0.2 | 0.3 |
| | 51.1 | 100% |

* To obtain these products the LHSV was 0.51 $h^{-1}$, the reaction temperature was 260°C and the reaction pressure was 4.14 bar (60 psig). Hydrogen gas was not supplied to the reactor to obtain the results of this Table. Water formed in the reaction was not considered a product in the ester selectivity calculation.

TABLE 5

| Products* | Weight %** | Eff. % |
|---|---|---|
| Ethyl acetate | 25.16 | 96.0 |
| Acetic acid | 0.03 | |
| Butanol+Butanal | 0.58 | |
| Ethyl ether | 0.05 | |
| Water | 0.40 | |
| | 26.22% | |

* To obtain these products the LHSV was 0.625 $h^{-1}$, the reaction temperature was 232°C, the reaction pressure was 4.14 bar (60 psig), and hydrogen gas was supplied to the reactor at 72 standard liters per hour, or 1.64 moles of hydrogen gas per mole of ethanol feed.

** The conversion results exclude about 1 percent acetaldehyde which was recycled for further reaction.

TABLE 6

| Products* | Weight %** | Eff. % |
|---|---|---|
| Ethyl acetate | 9.41 | 92.2 |
| Acetic acid | 0.17 | |
| Butanol+Butanal | 0.38 | |
| Ethyl ether | 0.12 | |
| Water | 0.12 | |
| | 10.20% | |

* To obtain these products the LHSV was 0.64 $h^{-1}$, the reaction temperature was 231°C, the reaction pressure was 4.2 bar (61 psig), and hydrogen gas was supplied to the reactor at 41 standard liters per hour.

** The conversion results exclude about 1.5 percent acetaldehyde which was recycled for further reaction.

This example illustrates the secondary reaction products and concentrations obtained from the method of this invention. The acetaldehyde obtained can be recycled at lower temperatures to obtain lower conversion results with better ethyl acetate selectivity. Of note, the highest mole feed ratio of hydrogen gas to alcohol feed is illustrated in this example. This example demonstrated the highest efficiency of ester production of Examples 1 through 4.

Example A .

This example illustrates the conversion of ethanol to ethyl acetate without increased hydrogen gas in the feed to the reactor. The procedure of this example was the same as for Example 1 except that a smaller catalyst screening reactor was used. The reactor contained 20 $cm^3$ of 3.175×3.175 mm Calsicat E-113TU copper chromite catalyst. Twelve liters per hour of nitrogen gas was fed to the reactor in this example. The analysis of the reaction products is presented in Table 7 and 8.

TABLE 7

| Products* | Weight % | Eff. % |
|---|---|---|
| Ethyl acetate | 14.9 | 68.6 |
| Acetaldehyde | 4.2 | 19.4 |
| Butanol+Butanal | 2.6 | 12.0 |
| | 21.7 | 100.0 |

* To obtain these products the LHSV was 1.0 $h^{-1}$, the reaction temperature was 221°C, and the reaction pressure was 3.52 bar (51 psig). Water formed in the reaction was not considered a product in the ester selectivity calculation.

TABLE 8

| Products* | Weight % | Eff. % |
|---|---|---|
| Ethyl acetate | 14.4 | 51.4 |
| Acetic acid | 0.2 | 0.7 |
| Acetaldehyde | 8.5 | 30.4 |
| Butanol+Butanal | 4.8 | 17.1 |
| Acetone | 0.1 | 0.4 |
| | 28.0 | 100.0 |

* To obtain these products the LHSV was 1.0 $h^{-1}$, the reaction temperature was 249°C, and the reaction pressure was 3.45 bar (50 psig). The material balance was:

$$\frac{\text{weight of products collected}}{\text{weight of reactants fed}}=99.3\%$$

This Example when compared to Example 4 demonstrates that a lower efficiency of ester production is obtained without regulating the mole ratio of hydrogen gas to ethanol in the reactor.

Example B and C

These examples illustrate that alkanols other than ethanol can be converted to their corresponding alkanoate esters with a copper chromite catalyst. The procedure of these examples was the same as for Eample A. The mole ratio of hydrogen gas to alkanol feed solution was not regulated in these examples. The analysis of the reaction products of Examples B and C is in Tables 9 and 10 respectively.

TABLE 9

| Products* | Weight % | Eff. % |
|---|---|---|
| Propyl propionate | 5.7 | 26.0 |
| Propanal | 12.3 | 56.2 |
| 2-Methyl pentanal | 1.5 | 6.9 |
| 2-Methyl Pentanol | 1.9 | 8.7 |
| Unknown | 0.5 | 2.2 |
| | 21.9 | 100.0 |

* To obtain these products the LHSV was 1.0 $h^{-1}$, the reaction temperature was 247°C, and the reaction pressure was 3.45 bar (50 psig). If the propanol were assumed to be recycled the calculated propyl propionate selectivity is 59.4%.

TABLE 10

| Products* | Weight % | Eff. % |
|---|---|---|
| Butyl butanoate | 6.6 | 26.4 |
| Butanal | 16.3 | 65.2 |
| 2-Ethyl hexanal | 0.5 | 2.0 |
| 2-Ethyl hexanol | 1.0 | 4.0 |
| Unknown | 0.6 | 2.4 |
| | 25.0 | 100.0 |

* To obtain these products the LHSV was 1.0 $h^{-1}$, the reaction temperature was 247°C, and the reaction pressure was 3.45 bar (50 psig).

These examples demonstrate the ability of a copper containing catalyst to convert primary alkanols to their corresponding esters.

Example 5

This example demonstrates the increased efficiency and productivity of the conversion of ethanol to ethyl acetate using the Harshaw Cu-0203 catalyst in operation from just after initiation of the reaction to about 750 hours of catalyst operation. In this example, ethanol is converted to ethyl acetate by the same procedure used in Example 1. Samples 1 to 9 are test results without regulation of the hydrogen gas ethanol mole ratio in the reactor and are for comparison with samples 10 to 18. Samples 10 to 18 are test results whereby the mole ratio of hydrogen gas to ethanol in the reactor was regulated at different flow rates, reactor temperatures, and reactor pressures. The test conditions and results of this experiment are in Table 11.

TABLE 11

| Sample | LHSV hour | Temp. °C | Pressure bar (psig) | Wt% AcH | $H_2$ Moles** per hour | Products* AcH | $H_2O^+$ | Acet | AcOH | $Et_2O$ | BuAl | BuOH | EtOAc | % Eff. (EtOAc) |
|---|---|---|---|---|---|---|---|---|---|---|---|---|---|---|
| 1 | 1.01 | 241 | 6.21 (90) | 5.00 | — | 3.41 | 1.94 | 0.00 | 0.20 | 0.71 | 1.08 | 2.27 | 24.01 | 79.5 |
| 2 | 1.57 | 261 | 4.14 (60) | 10.00 | — | 7.66 | 3.23 | 0.12 | 0.60 | 0.60 | 2.74 | 3.75 | 23.69 | 68.2 |
| 3 | 1.50 | 259 | 8.28 (120) | 10.00 | — | 5.37 | 3.03 | 0.19 | 0.50 | 0.99 | 1.59 | 4.05 | 23.22 | 69.2 |
| 4 | 1.47 | 221 | 8.35 (121) | 0.01 | — | 2.75 | 0.67 | 0.00 | 0.05 | 0.21 | 0.39 | 0.74 | 12.02 | 85.3 |
| 5 | 0.99 | 222 | 4.14 (60) | 10.00 | — | 3.47 | 1.30 | 0.00 | 0.09 | 0.64 | 0.73 | 1.35 | 16.45 | 80.0 |
| 6 | 1.00 | 221 | 8.35 (121) | 10.00 | — | 4.07 | 1.54 | 0.00 | 0.18 | 0.69 | 0.52 | 2.13 | 12.41 | 71.0 |
| 7 | 1.00 | 243 | 6.28 (91) | 5.00 | — | 3.67 | 2.10 | 0.02 | 0.24 | 0.95 | 1.11 | 2.49 | 23.93 | 77.6 |
| 8 | 0.96 | 250 | 4.21 (91) | 0.01 | — | 3.21 | 2.22 | 0.06 | 0.50 | 0.75 | 1.44 | 2.24 | 30.95 | 81.1 |
| 9 | 0.50 | 262 | 4.2 (61) | 0.00 | — | 3.37 | 3.66 | 0.18 | 0.63 | 1.12 | 2.56 | 2.80 | 40.07 | 78.5 |
| 10 | 0.81 | 251 | 2.14 (31) | 0.00 | 0.45 | 6.30 | 2.98 | 0.25 | 0.37 | 0.36 | 3.19 | 2.39 | 27.7 | 74.4 |
| 11 | 0.83 | 252 | 2.07 (60) | 0.00 | 0.45 | 4.00 | 2.32 | 0.13 | 0.29 | 0.54 | 2.08 | 2.09 | 30.76 | 80.5 |
| 12 | 0.76 | 232 | 1.035 (30) | 0.00 | 0.45 | 4.26 | 1.39 | 0.10 | 0.13 | 0.20 | 1.70 | 1.17 | 23.58 | 83.4 |
| 13 | 0.79 | 232 | 4.2 (61) | 0.00 | 0.45 | 2.80 | 1.26 | 0.00 | 0.10 | 0.32 | 1.08 | 1.07 | 24.62 | 86.6 |
| 14 | 0.63 | 241 | 3.17 (46) | 0.00 | 0.45 | 3.40 | 1.91 | 0.10 | 0.19 | 0.41 | 1.83 | 1.55 | 30.0 | 83.4 |
| 15 | 0.60 | 242 | 3.10 (45) | 0.00 | 1.61 | 2.40 | 1.29 | 0.12 | 0.11 | 0.22 | 1.42 | 0.64 | 31.59 | 89.3 |
| 16 | 0.97 | 242 | 5.17 (75) | 0.01 | 1.61 | 2.66 | 1.12 | 0.05 | 0.11 | 0.34 | 1.05 | 1.01 | 26.79 | 88.0 |
| 17 | 0.68 | 241 | 5.17 (75) | 0.00 | 3.21 | 1.59 | 0.99 | 0.04 | 0.07 | 0.22 | 0.79 | 0.46 | 30.23 | 92.1 |
| 18 | 0.58 | 240 | 5.24 (76) | 0.01 | 4.15 | 1.05 | 0.91 | 0.02 | 0.04 | 0.09 | 0.55 | 0.21 | 27.22 | 94.1 |

* Products are in weight percent
** Flow rates of hydrogen gas vary in this example. Consistent flow rates would alter the efficiency calculation.
\+ This reading is not adjusted for condensate water from the dry ice traps. Humidity is not controlled in this example. Humidity can affect this value. Approximately 0.5 percent condensate water is present in all values. No correction for water was made in the ester efficiency calculations.

This example illustrates the results of continued operation of the method of this invention using a copper chromite catalyst. Comparison of samples 10 and 12 demonstrates that a decrease in reactor temperature with a constant hydrogen gas flow rate and reactor pressure increases the efficiency of ester production, but decreases the yield. Comparison of samples 10 to 11 and samples 12 to 13 illustrates an increase in the efficiency of ester production and total ester produced with increased reactor pressure at about 250°C and about 230°C, respectively. Comparison of samples 15 to 16 illustrates a relatively steady efficiency of ester production, but a decrease in total ester produced with increased reactor pressure to 5.175 bar (75 psig). Comparison of samples 16, 17, and 18 illustrate an increase in efficiency of ester production with an increase in hydrogen gas feed rate at a constant reactor temperature and reactor pressure.

Example 6

This example demonstrates the conversion of Isobutanol to isobutyl isobutyrate ester with hydrogen gas in the feed to the reactor. In this example the alkanol is converted to the alkanoate ester by the same procedure used in Example 1 except that 180 $cm^3$ of 3.175×3.175 mm United Catalyst Inc T-1990 12 percent copper oxide on alumina catalyst is used to obtain the reaction products. The analysis of the reaction products is in Table 12.

TABLE 12

| Products* | Weight % | Eff. %** |
|---|---|---|
| Isobutyl isobutarate | 5.5 | 37.7 |
| Water | 2.1 | 14.4 |
| Isobutyraldehyde | 3.4 | 23.3 |
| Lights | 3.6 | 24.6 |
| | 14.6 | 100.0 |

* To obtain these products the LHSV was 1.0 $h^{-1}$, the reaction temperature was 250°C, the reaction pressure was 4.14 bar (60 psig), and hydrogen gas was supplied to the reactor at 2.0 moles per hour.

** The calculation of efficiency percent assumes that the aldehyde is a product.

Although the alumina support caused significant dehydration of isobutanol, as evidenced by the water and lights formed, little if any aldehyde condensation products were formed. This example demonstrates that a copper containing catalyst, other than a copper chromite catalyst, functions with the method of this invention.

Example D

This example demonstrates the increased productivity of ethyl acetate in ethanol conversion when acetaldehyde is substituted for part of the ethanol feed solution. In this example, ethanol is converted to ethyl acetate by the same procedure used in Example 1 except that 180 cc of a 4.8×2.38 mm United Catalysts Inc. G-66B copper oxide/zinc oxide catalyst is used. The test conditions and results of this example are in Table 13.

TABLE 13

| Sample | LHSV hour | Reaction temp°C | Pressure bar (psig) | Feed AcH% | Products | | | | | | Eff.%+ |
|---|---|---|---|---|---|---|---|---|---|---|---|
| | | | | | Water | AcH | Misc** | BuAl | BuOH | EtOAc | |
| 1 | 1.40 | 254 | 6.00 (87) | 34 | 2.0 | 10.4 | 0.9 | 3.2 | 2.1 | 22.8 | 73.6 |
| 2 | 1.33 | 252 | 6.00 (87) | 0 | 0.8 | 5.8 | 0.5 | 1.4 | 0.2 | 13.5 | 82.3 |
| 3 | 0.67 | 213 | 6.14 (89) | 34 | 2.6 | 5.8 | 0.3 | 2.1 | 2.9 | 18.0 | 69.5 |
| 4 | 1.0 | 270 | 6.07 (88) | 51 | 4.0 | 6.4 | 2.0 | 3.9 | 3.8 | 40.4 | 66.8 |

* Product values are in weight percent.
** Miscellaneous products include ether, acetone, and acetic acid.
\+ The efficiency calculation includes water, but assumes the recycling of acetaldehyde.

After about one week of operation the tubular reactor became plugged with a white solid which was analyzed to be zinc acetate. The zinc acetate was presumably formed when the by-product, acetic acid, reacted with the zinc oxide in the catalyst. This is more severe when 190 proof ethanol is used as a feed solution. This is because the increased water content of the feed solution causes an increase in acetic acid formation.

This example demonstrates that an increase in the acetaldehyde concentration with this catalyst system leads to an increase in ethyl acetate production.

Example 7

This example demonstrates the lower productivity of ethyl acetate ester in a system using a copper chromite catalyst and wherein hydrogen gas is removed from the reaction zone. The hydrogen gas is removed or regulated in the reaction zone by replacing some ethanol in the feed solution with acetaldehyde. In this example, ethanol is converted to ethyl acetate by the same procedure used in Example 1. A 180 $cm^3$ 3.175×3.175 mm Harshaw Cu-0203 copper chromite catalyst is used in this reaction. The test conditions and results of this experiment are in Table 14.

TABLE 14

| Sample | Feed AcH% | AcH | Water | Products* $Et_2O$ | Misc | BuAl | BuOH | EtOAc | EtOAc Eff %** |
|---|---|---|---|---|---|---|---|---|---|
| 1 | 0 | 4.5 | 3.0 | 0.9 | 1.4 | 1.4 | 1.9 | 22.8 | 72.6 |
| 2 | 34 | 19.1 | 6.6 | 0.4 | 1.6 | 4.0 | 8.4 | 11.0 | 34.4 |

* The product values are in weight percent. The LHSV was 1.0 $h^{-1}$, the reaction temperature was 251°C, and the reaction pressure was 6.3 bar (91 psig).

** The efficiency calculation assumes that acetaldehyde is recycled.

The comparison of the results of this example with the results of Example B illustrate that a zinc containing catalyst has an increased production of ester wherein the concentration of aldehyde in the reaction zone is increased. An increased concentration of aldehyde in the reaction zone of a copper chromite catalyst decreases ester production. These examples suggest that their reaction pathways to ester production are different.

**Claims**

1. A method for producing an ester by reacting a primary $C_1$—$C_{15}$ alkanol in vapor phase at a temperature of 200 to 300°C and a pressure of 0 to 34.5 bar in the presence of a dehydrogenation catalyst, and recovering the ester characterized in that the catalyst is a copper chromite catalyst, optionally containing barium oxide and that the mole ratio of hydrogen and alkanol is maintained in the range of 0.4 to 5 moles $H_2$, preferably 0.6 to 3 moles $H_2$ to 1 mole alkanol.

2. The method according to Claim 1, characterized in that the recovering of the ester is achieved by azeotropic distillation.

3. The method according to Claim 1 or 2, characterized in that excess hydrogen is recycled to the reaction.

**Patentansprüche**

1. Verfahren zur Herstellung eines Esters durch Umsetzung eines primären $C_1$- bis $C_{15}$-Alkanols in Dampfphase bei einer Temperatur von 200 bis 300°C unter einem Druck von 0 bis 34,5 bar in Gegenwart eines Dehydrierungskatalysators und Gewinnen des Esters, dadurch gekennzeichnet, daß man als Katalysator einen Kupferchromit-Katalysator verwendet, gegebenenfalls enthaltend Bariumoxid, und daß das Molverhältnis Wasserstoff zu Alkanol bei 0,4:5 mol $H_2$, vorzugsweise 0,6 bis 3 mol $H_2$ je mol Alkanol gehalten wird.

2. Verfahren nach Anspruch 1, dadurch gekennzeichnet, daß man den Ester durch azeotrope Destillation gewinnt.

3. Verfahren nach Anspruch 1 oder 2, dadurch gekennzeichnet, daß überschüssiger Wasserstoff in die Reaktion rückgeleitet wird.

**Revendications**

1. Procédé de production d'un ester par réaction d'un alcanol primaire en $C_1$ et $C_{15}$ en phase vapeur à une température de 200 à 300°C et à une pression de 0 à 34,5 bars en présence d'un catalyseur de

déshydrogénation, et séparation de l'ester, caractérisé en ce que le catalyseur est un catalyseur au chromite de cuivre, contenant facultativement de l'oxyde de baryum, et en ce que le rapport molaire de l'hydrogène à l'alcanol est maintenu dans l'intervalle de 0,4 à 5 moles de $H_2$, de préférence de 0,6 à 3 moles de $H_2$, par mole d'alcanol.

2. Procédé selon la revendication 1, caractérisé en ce que la séparation de l'ester est effectuée par distillation azéotropique.

3. Procédé selon la revendication 1 ou 2, caractérisé en ce que l'hydrogène en excès est recyclé dans la réaction.

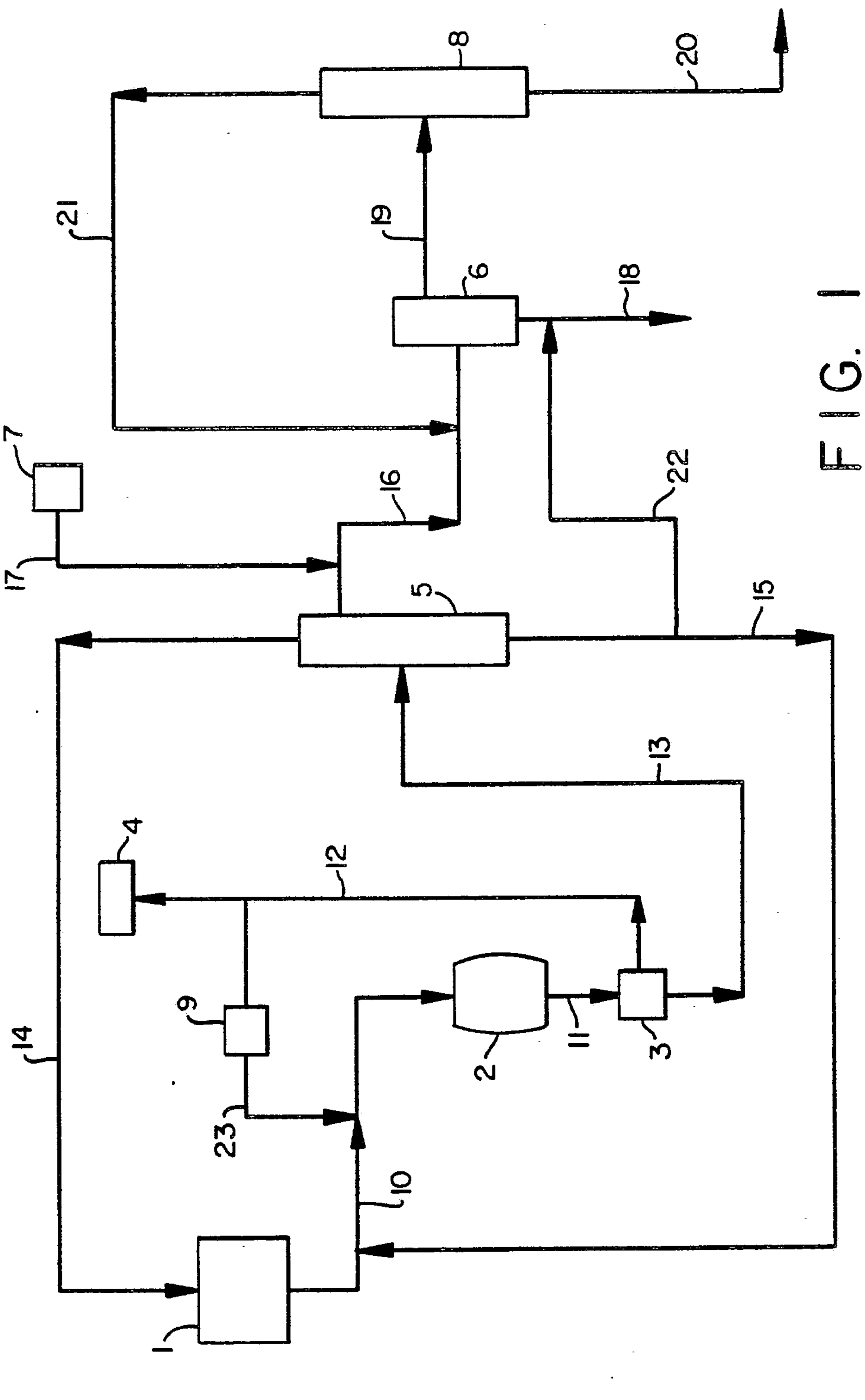


FIG. 1

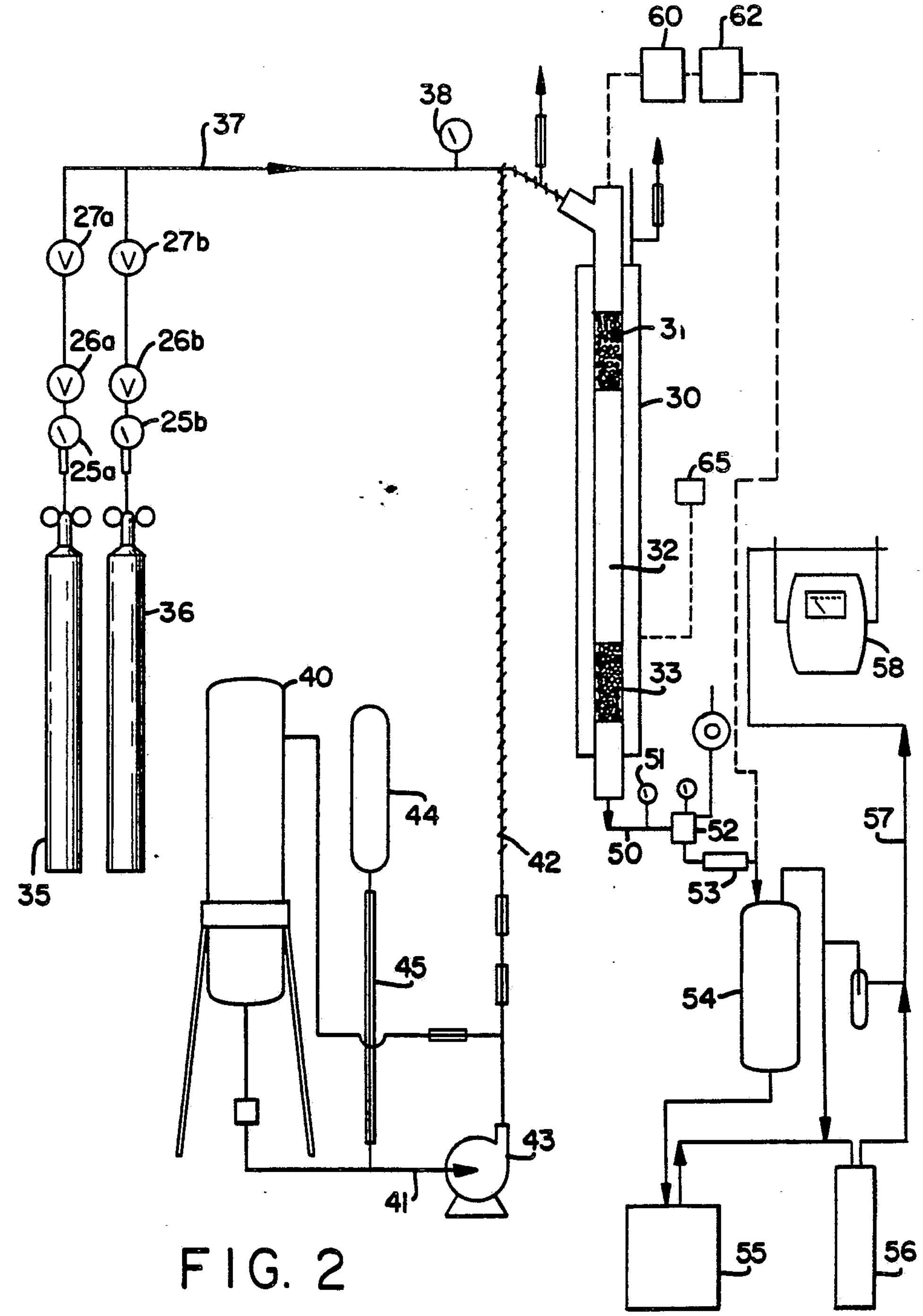
60
62
38
37
27a
27b
26a
26b
25a
25b
31
30
65
32
36
58
33
40
44
51
52
50
57
42
35
53
45
54
43
41
55
56

FIG. 2